# EUROPEAN PATENT SPECIFICATION

(11) **EP 4 422 476 B1**
(45) Date of publication and mention of the grant of the patent: **15.04.2026**
(21) Application number: 22814544.7
(22) Date of filing: 28.10.2022
(51) Int. Cl.: A61B 5/00

(54) **SHIELDING CAP FOR DERMATOSCOPES FITTED WITH MAGNETS TO BE USED TO EXAMINE PATIENTS WITH CARDIAC IMPLANTABLE ELECTRONIC DEVICES**
SCHUTZKAPPE FÜR MIT MAGNETEN VERSEHENE DERMATOSKOPE ZUR UNTERSUCHUNG VON PATIENTEN MIT IM HERZEN IMPLANTIERBAREN ELEKTRONISCHEN VORRICHTUNGEN
CAPUCHON DE PROTECTION POUR DERMATOSCOPES ÉQUIPÉS D'AIMANTS À UTILISER POUR EXAMINER DES PATIENTS AVEC DES DISPOSITIFS ÉLECTRONIQUES CARDIAQUES IMPLANTABLES

(30) Priority: 28.10.2021 PL 43934921
(43) Date of publication of application: 04.09.2024
(73) Proprietor: Gdanski Uniwersytet Medyczny, 80-210 Gdansk (PL); Politechnika Gdanska, 80-233 Gdansk (PL)
(72) Inventor: USAREK, Zbigniew, 80-283 Gdansk (PL); SLAWINSKI, Grzegorz, 80-288 Gdansk (PL); SLAWINSKA, Martyna, 80-288 Gdansk (PL); SOBJANEK, Michal, 80-034 Gdansk (PL); NOWICKI, Roman J., 81-784 Sopot (PL); LEWICKA, Ewa, 80-283 Gdansk (PL); RACZAK, Grzegorz, 80-447 Gdansk (PL)
(74) Representative: Godlewski, Piotr
(86) International application number: PCT/PL2022/050073
(87) International publication number: WO 2023/075622

(56) References cited:
- FR-A1- 3 013 579
- JP-A- 2002 065 869
- US-A1- 2021 137 633
- US-A1- 2021 219 842
- SLAWINSKI GRZEGORZ ET AL: "Electromagnetic Field Associated With Dermoscope Magnets May Affect the Safety of Cardiac Implanted Electronic Devices Patients", FRONTIERS IN CARDIOVASCULAR MEDICINE, vol. 8, 1 January 2021 (2021-01-01), pages 757032, XP093016782, Retrieved from the Internet <URL:https://www.ncbi.nlm.nih.gov/pmc/articles/PMC8551606/pdf/fcvm-08-757032.pdf> DOI: 10.3389/fcvm.2021.757032

## Description

### Technical Field

The invention concerns a shielding cap for dermatoscopes fitted with magnets to be used to examine patients with cardiac implantable electronic devices (CIED).

### Background Art

A dermoscope (dermatoscope) is a diagnostic device designed so as to ensure precise examination of the skin lesions on patient's skin.

Modern dermatoscopes are characterised by ergonomic construction and mobility-ensuring size, thanks to which examination can be performed in various conditions irrespective of the location of the skin lesion. In addition, the devices can be linked to a smartphone, which enables documenting the examined lesion by taking precise photographs.

Previously known in state of the art there are many solutions concerning modern dermatoscopes, e.g. in patent applications: CN211484516U, CN212281337U, US2014066727A1, US10617305B2, and CN212234422U.

Patent application WO2016205950A1 concerns a skin imaging device, in particular one which is used in combination with a mobile device for obtaining digital images using a digital camera and the source of light ensured by the mobile device. In one of its variants, the device is fitted with a ferromagnetic element, especially a permanent magnet, which enables quick mounting of the device on the mobile device casing. The device in the described variant constitutes the source of magnetic field.

Patent PL/EP2991543T3 discloses a system for visual representation of body areas, in particular an adapter used for medical purposes, the main role of which is to ensure a portable visual representation system which employs the source of light of the electronic recording device as the lighting source and which provides a portable digital recording element thanks to a specific configuration of converging lenses with an appropriate diopter mounted on the front side of the camera.

The scientific article titled "Electromagnetic Field Associated With Dermoscope Magnets May Affect the Safety of Cardiac Implanted Electronic Devices Patients" (Slawinski Grzegorz ET AL, 2021-01-01, DOI: 10.3389/fcvm.2021.757032) and the patent applications FR 3 013 579 A1 (PIXIENCE, 2015-05-29), US 2021/137633 A1 (MULLANI ET AL, 2021-05-13) discloses and US 2021/219842 A1 (TROTZENBERG ET AL, 2021-07-22) provide pertinent information regarding the background of the invention. The said scientific article discloses a disposable clear plastic cap mounted on a dermatoscope. The applications FR 3 013 579 A1 and US 2021/137633 A1 disclose dermatoscopes with built-in magnets. US 2021/219842 A1 discloses a magnetic screen made of soft material.

In addition to the traditional use in the diagnostics of skin cancers, the dermatoscope is currently extensively used as a supporting tool in general dermatology. Therefore, it happens ever more frequently that patients with cardiac implantable electronic devices (CIED) are subject to examination using the dermatoscope.

Some of the dermatoscopes available in the market are fitted with built-in magnets enabling precise and quick assembly of the front plate of a dermatoscope. Magnetic field generated by the magnets may disturb the functioning of the CIED during a dermatoscope examination in the group of patients referred to above [A. Rishpon and others, Assessment of the Safety Risk of Dermatoscope Magnets in Patients with Cardiovascular Implanted Electronic Devices, JAMA Dermatol. 2018, 1, 154 (10):1204-1207].

Despite the fact that the dermatoscopes featuring built-in magnets are the source of a strong magnetic field and may potentially threaten the health of a patient with the CIED, there are no solutions known which would enable safe examination of such a patient with a dermatoscope.

The purpose of the invention is to provide a dermatoscope cap which would enable shielding of the magnetic field, thus ensuring reduction of the magnetic induction of a dermatoscope to the limits defined by CIED manufacturers and enabling safe examination of skin lesions with a dermatoscope containing built-in magnet, located in the vicinity of the CIED pocket.

### Summary of Invention

The invention concerns a cap for mounting on a dermatoscope 2, the dermatoscope featuring built-in magnets 3 keeping a dermatoscope's contact plate 4 in place, wherein the cap 1 is composed of a magnetic screen 5 made of a magnetically soft material, and at least one protective casing 6,7,8, wherein the magnetically soft material has maximum relative magnetic permeability (µ) falling in the range 10³-10⁷, thanks to which the magnetic screen 5 reduces the magnetic induction occurring above the contact plate of a dermatoscope.

In the solution according to the invention the thickness of the magnetic screen 5 is selected individually to a specific dermatoscope model, so that the maximum value of the induction measured at the surface of the cap 1 mounted on the dermatoscope 2 does not exceed 0.5 mT. Preferably, the thickness of the magnetic screen 5 does not exceed 1 mm, so as to ensure that contact between the contact plate 4 of the dermatoscope and the patient's skin is retained.

In accordance with the invention, the magnetically soft material preferably is an alloy of metals on the basis of iron, nickel, or cobalt (e.g. permalloy, permendur).

In the solution according to the invention, in one invention embodiment the cap 1 is composed of a magnetic screen 5 protected with a protective casing 6, where the protective casing 6 is made of plastic. Preferably, the protective casing 6 is composed of two parts.

In another invention embodiment the protective casing comprises external protective casing 7 and internal protective casing 8, where the protective casings 7, 8 are made of elastomer.

Because of the simple structure of the cap 1, the solution according to the invention ensures the possibility to adjust the cap 1 to many dermatoscope models of various diameters of the contact plate 4 and various range of the dermatoscope working field, thanks to which it can be universally used in various dermatoscopes.

The cap 1 according to the invention is designated for shielding the magnetic field generated by the magnets 3 used for mounting the contact plate glass 4 on the dermatoscope 2.

The solution according to the invention solves the problem of availability of safe dermatoscopic examination for patients with the CIED, especially in the case of dermatoscopes fitted with magnets which basically cannot be used to examine patients with the CIED. Therefore, the invention also concerns the use of the cap 1 according to the invention in a dermatoscope so as to perform dermatoscopic examination of skin lesions in patients with cardiac implantable electronic devices (CIED).

### Brief Description of Drawings

The object of the invention is illustrated in its embodiments and on a drawing, where:

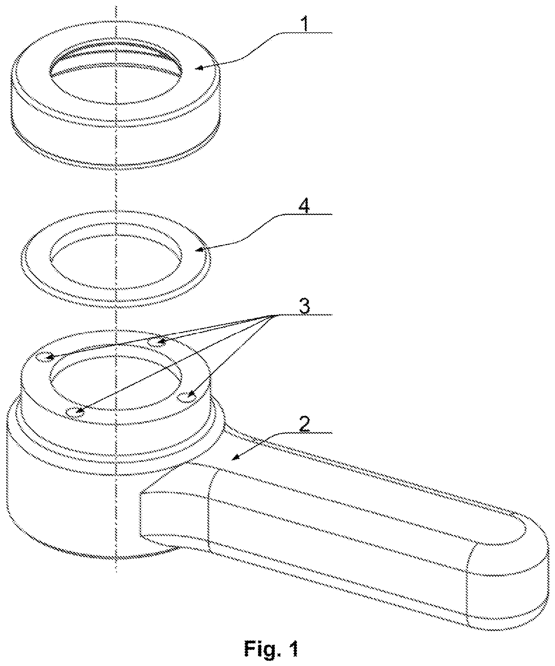

[Fig.1] presents a perspective view of a dermatoscope with a shielding cap, where the individual numerical references are as below: 1-shielding cap, 2- dermatoscope, 3-mounting magnets, 4- contact plate glass
[Fig.2] shows a schematic representation of the first embodiment variant of the shielding cap 1 viewed from the side of the patient's skin;
[Fig.3] presents a cross section A-A of the cap depicted in Fig. 2 in the scale 2:1,
   where individual numerical references are as follows: 5- magnetic screen, 6-screen protective casing;
[Fig.4] presents a schematic representation of the second embodiment variant of the shielding cap 1 viewed from the side of the patient's skin;
[Fig.5] presents a cross section B-B of the cap depicted in Fig. 4 in the scale 2:1, where individual numerical references are as follows: 5- magnetic screen, 7-external protective casing of the screen, 8- internal protective casing of the screen;
[Fig.6] presents a schematic representation of the third embodiment variant of the shielding cap 1 mounted on a dermatoscope 2 viewed from the side of the patient's skin;
[Fig.7] presents a cross section of the shielding cap 1 in the fourth embodiment variant, where individual numerical references are as follows: 5- magnetic screen, 7-external protective casing of the screen, 8- internal protective casing of the screen;
[Fig.8-9] show measurements of the magnetic induction distribution above the contact plate of a dermatoscope without the shielding cap versus measurements taken following its mounting on the contact plate of a dermatoscope.

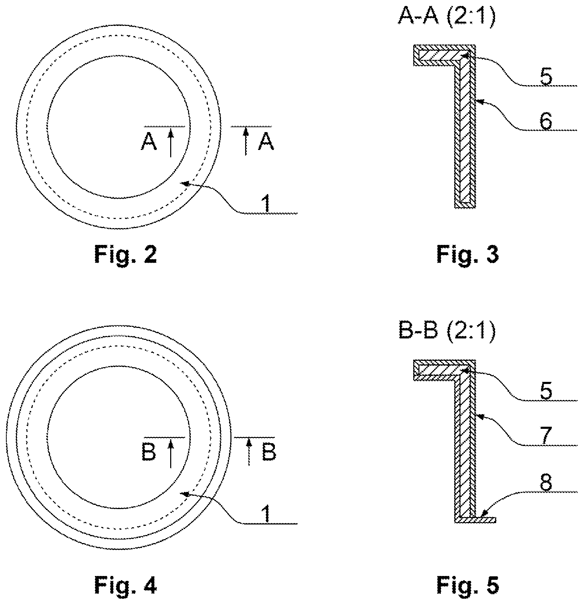

In accordance with the variant presented in embodiment 1 and in Fig. 2 and 3, the shielding cap **1** is fitted with a protective casing **6** made of plastic of low thermal conductivity not in excess of 1 W/(m*K), preferably of silicone. In one of the invention variants the protective casing **6** is composed of two parts.

In another variant of the invention presented in embodiment 2 and in Fig. 4 and 5, the shielding cap **1** is composed of a magnetic screen **5** protected with an external protective casing **7** and internal protective casing **8,** where the protective casings **7, 8** are made of elastomer.

The protective casings **6,7** and **8** play the role of an element protecting the dermatoscope. The protective casings **6** and **8** protect the dermatoscope **2** against scratching which could occur in direct contact of the dermatoscope with the magnetic screen **5.** In addition, the protective casings **6, 7** and **8** protect the screen **5** from the impact of external conditions. Moreover, the protective casings **6** and **7** raise the comfort of the patient subject to dermatoscopic examination by eliminating direct contact of the metal magnetic screen **5** with the patient's skin. In addition, if the screen is made of alloys containing nickel, the protective casings **6** and **7** protect the patient from allergic reaction which might occur as the result of direct contact of such an alloy with the skin of the patient subject to examination. In accordance with the invention, the protective casings 6 and 7 may be disposable, playing the protective function and ensuring hygiene of the examination, and thanks to the fact that they can be produced of biodegradable materials their use does not burden the natural environment with generation of hard to dispose waste. Another advantage of the solution according to the invention is that the shielding cap 1 may be a reusable and easily disinfectable product, disinfection of which does not, thanks to its structure, affect the quality of the dermatoscopic image or the patient's comfort during the examination.

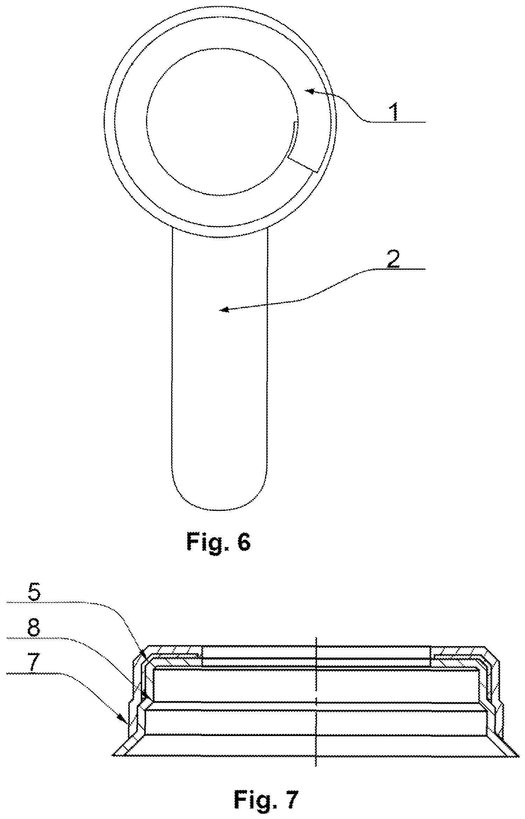

It is a known fact that various models of dermatoscopes may differ in the dimensions of the dermatoscope contact plate, i.e. the diameter of the contact plate glass **4.** Therefore, in one of the variants of the solution according to the invention the shielding cap 1 is cut through its circumference, as illustrated in Fig.6. Thanks to the cut it is possible to adjust the cap 1 to a dermatoscope of various diameters of the contact plate glass **4** without limiting the dermatoscope examination field, which makes it possible for the solution according to the invention to find universal application in various dermatoscopes.

The solution according to the invention is illustrated in its embodiments which do not limit its scope.

### Examples

### Example 1

In the first variant of the invention shown in Fig. 2 the cap 1 is given the shape of a ring of the outer diameter of 40 mm. The presence of an opening of the diameter of 33 mm in the presented embodiment of the cap 1 enables undisturbed observation of the patient's skin. The cross-section A-A of the cap 1 is presented in Fig.3 in double enlargement. The cross section is a reversed letter L in shape. The cap 1 is 8 mm high and 2 mm thick. The cap 1 is composed of a magnetic screen 5 1 mm thick, made of permendur. Because of the substantial thickness of the screen and the high saturation induction of permendur, which may reach 2.43 T, the cap in this embodiment variant may be preferably used in dermatoscopes the magnetic induction of which exceeds 5 mT when measured at the surface of the contact plate. In accordance with this variant of the solution, the screen 5 is sunk in a silicone protective casing 6, which protects the dermatoscope 2 from scratching and thanks to its low thermal conductivity which can reach 0.14 W/(m*K) it reduces the patient's thermal discomfort accompanying the examination. Since the protective casing 6 can be disinfected, the cap 1 is a reusable product.

### Example 2

In the second embodiment of the invention presented in Fig. 4 the cap 1 is given the shape of a ring of the inner diameter of 33 mm, which ensures undisturbed observation of the patient's skin. The cross-section B-B of the cap 1 is presented in Fig.5 in double enlargement. The cap 1 is composed of a magnetic screen 5 1 mm thick made of permalloy, the external protective casing 7 and internal protective casing 8.

The distance between the magnetic screen 5 and the dermatoscope is ensured by the internal protective casing 8 made of polypropylene which protects the dermatoscope from scratching which might occur in the case of a direct contact between the dermatoscope and the magnetic screen 1.

The external protective casing 7 also made of polypropylene keeps the magnetic screen 5 in the pre-set position and protects it from the impact of external conditions. Another role of the protective casing 7 is to reduce the thermal discomfort of the patient subject to dermatoscopic examination thanks to the low thermal conductivity of polypropylene amounting to ca. 0.11 W/(m*K).

The advantage of the cap 1 in the embodiment variant described above is the possibility to dismount its individual components, which in turn enables replacement of the magnetic screen. In practice, such a solution makes it possible to adjust the thickness of the screen 5 within the range from 0.1 to 1 mm or replace the material the screen is made of so as to adjust it to various dermatoscopes, which generally generate magnetic field of various induction levels. Moreover, the possibility to disassemble the cap 1 in this particular embodiment variant to individual components makes it possible to quickly replace the protective casing 7, and if the cap needs to be disposed of, the disposal is simpler and the recycling of the material the magnetic screen 5 is made of is ensured.

### Example 3

In the third variant of the invention presented in Fig.6 the cap 1 is fitted with a magnetic screen 5 0.2 mm thick, enclosed in a protective casing made of elastomer. The cap 1 is characterised in that it is cut through its circumference. In the embodiment presented in Fig. 6 the inner diameter of the cap 1 is 39 mm, while the diameter of the contact plate of the dermatoscope 2 is 36 mm. Despite the difference in the dimensions given above, the cap 1 may be adjusted to fit the dermatoscope 2 thanks to the presence of the cut and its own high elasticity.

In this variant of the invention the protective casing may be either a single-piece element (protective casing 6), like in embodiment 1, or can be made of two parts, like in embodiment 2 (protective casings 7,8).

### Example 4

In the fourth embodiment variant presented in Fig .7 the cap 1 was designed to fit a specific model of dermatoscope, namely Dermlite DL4. The magnetic screen 5 0.1 mm thick was made of a magnetically soft alloy called MUMETALL^{®} obtained from VACUUMSCHMELZE GmbH & Co. KG. The protective casings, on the other hand, both the external protective casing 7, and the internal protective casing 8, were produced of polylactide using the 3D printing technique.

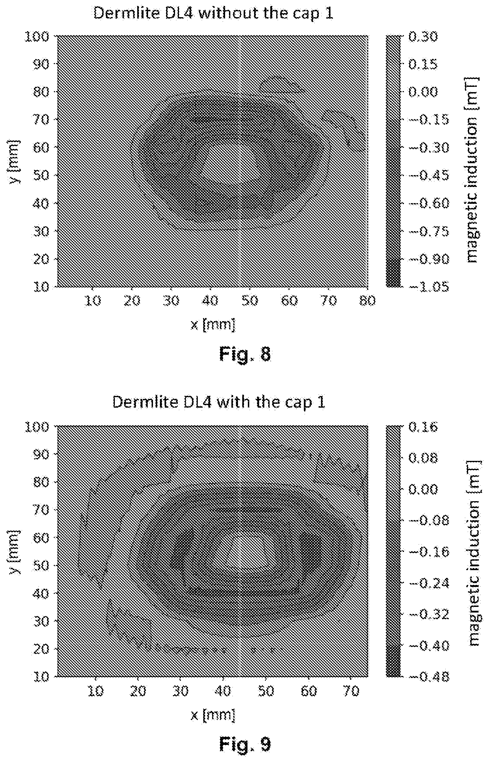

For this variant of the solution according to the invention the measurements of the magnetic induction distribution above the contact plate 4 of the dermatoscope 2 were performed without the shielding cap 1 and upon its mounting on the contact plate 4 of the dermatoscope 2. The measurements were taken using the MPR-H2 magnetic field meter produced by Mag-Lab s.c. The results obtained in both cases are presented, respectively, in Fig. 8 and Fig. 9.

In the case of a dermatoscope devoid of the cap the induction value of the identified magnetic field locally exceeded 1 mT. Following the mounting of the shielding cap 1, on the other hand, the maximum value of the recorded induction did not exceed 0.48 mT. Hence, it has been evidenced, that the solution according to the invention reduces the magnetic field by more than 50%. The obtained result allows to conclude that the shielding cap according to the invention makes it possible for dermatoscopes fitted with magnets to satisfy the requirements of manufacturers of the CIED devices as concerns safety of application of devices which emit magnetic field in the group of patients with cardiac implantable electronic devices.

## Claims

1. A cap for mounting on a dermatoscope (2), the dermatoscope featuring built-in magnets (3) keeping a dermatoscope' s contact plate (4) in place, wherein the cap (1) is composed of a magnetic screen (5) made of a magnetically soft material, and at least one protective casing (6,7,8), wherein the magnetically soft material has maximum relative magnetic permeability falling in the range 10³-10⁷, thanks to which the magnetic screen (5) reduces the magnetic induction occurring above the contact plate of the dermatoscope.

2. The cap according to Claim 1, wherein the thickness of the magnetic screen (5) is 1 mm or less.

3. The cap according to Claims 1-2, wherein the protective casing (6) is made of plastic.

4. The cap according to Claim 3, wherein the protective casing (6) is composed of two parts.

5. The cap according to Claims 1-3, wherein the protective casing comprises an external protective casing (7) and internal protective casing (8), where the protective casings (7, 8) are made of elastomer.

6. Use of the cap (1) as described in any of the Claims 1-5, on a dermatoscope to perform dermatoscopic assessment of skin lesions in patients with cardiac implantable electronic devices (CIED).

## Patentansprüche

1. Eine Kappe zur Befestigung an einem Dermatoskop (2), wobei das Dermatoskop über eingebaute Magnete (3) verfügt, die die Kontaktplatte (4) des Dermatoskops an ihrem Platz halten, wobei die Kappe (1) aus einem magnetischen Schirm (5) aus einem magnetisch weichen Material und mindestens einem Schutzgehäuse (6, 7, 8) besteht, wobei das magnetisch weiche Material eine maximale relative magnetische Permeabilität im Bereich von 10³ bis 10⁷ aufweist, wodurch der magnetische Schirm (5) die über der Kontaktplatte des Dermatoskops auftretende magnetische Induktion reduziert.

2. Die Kappe mach Anspruch 1, wobei die Dicke des magnetischen Schirms (5) 1 mm oder weniger beträgt.

3. Die Kappe nach Ansprüchen 1-2, wobei das Schutzgehäuse (6) aus Kunststoff besteht.

4. Die Kappe nach Anspruch 3, wobei das Schutzgehäuse (6) aus zwei Teilen besteht.

5. Die Kappe nach Ansprüchen 1 bis 3, wobei die Schutzhülle eine äußere Schutzhülle (7) und eine innere Schutzhülle (8) umfasst, wobei die Schutzhüllen (7, 8) aus Elastomer bestehen.

6. Verwendung der Kappe (1) nach einem der Ansprüche 1 bis 5 an einem Dermatoskop zur Durchführung einer dermatoskopischen Beurteilung von Hautläsionen bei Patienten mit implantierbaren elektronischen Herzgeräten.

## Revendications

1. Capuchon pour montage sur un dermatoscope (2), le dermatoscope comportant des aimants intégrés (3) maintenant la plaque de contact du dermatoscope (4) en place, dans lequel le capuchon (1) est composé d'un écran magnétique (5) fait d'un matériau magnétiquement doux, et d'au moins un boîtier de protection (6,7,8), dans lequel le matériau magnétiquement doux présente une perméabilité magnétique relative maximale comprise entre 10³-10⁷, grâce à laquelle l'écran magnétique (5) réduit l'induction magnétique se produisant au-dessus de la plaque de contact du dermatoscope.

2. Le bouchon selon la revendication 1, dans lequel l'épaisseur de l'écran magnétique (5) est de 1 mm ou moins.

3. Le capuchon selon les revendications 1 et 2, dans lequel le boîtier de protection (6) est en plastique.

4. Le capuchon selon la revendication 3, dans lequel le boîtier de protection (6) est composé de deux parties.

5. Le capuchon selon les revendications 1 à 3, dans lequel le boîtier de protection comprend un boîtier de protection externe (7) et un boîtier de protection interne (8), les boîtiers de protection (7, 8) étant en élastomère.

6. Utilisation du capuchon (1) selon l'une des revendications 1 à 5, sur un dermatoscope pour effectuer une évaluation dermatoscopique des lésions cutanées chez les patients porteurs de dispositifs électroniques implantables cardiaques.
